# EUROPEAN PATENT APPLICATION

(11) **EP 2 719 374 A1**
(43) Date of publication of application: **16.04.2014**
(21) Application number: 12188407.6
(22) Date of filing: 12.10.2012
(51) Int. Cl.: A61K 9/00, A61K 31/46, A61K 47/42

(54) **Drug delivery device**

(71) Applicant: Rheinisch-Westfälische Technische Hochschule (RWTH) Aachen, 52056 Aachen (DE); Spintec Engineering GmbH, 52066 Aachen (DE); Heinrich-Heine-Universität Düsseldorf, 40225 Düsseldorf (DE)
(72) Inventor: Haupt,, Meike, 52074 Aachen (DE); Rheinnecker, Michael, 53881 Euskirchen (DE); Wöltje, Michael, 52074 Aachen (DE); Breitkreutz, Jörg, 45721 Haltern am See (DE)
(74) Representative: Remus, Alvaro Johannes

(57) **Abstract**

The invention relates to a device and kit for introducing at least one physiologically active compound into an organ or cavity of a human or animal body. The device comprises at least one silk material and is loaded with the physiologically active compound, wherein the silk material comprises at least one cleavage site that is cleavable by at least one degrading agent. For example, the incorporation of cleavage sites 10 in the form of thinner regions compared to a ring-shaped silk material 20 allows for controlled destruction of the silk material into smaller elements 30.

## Description

### Background of the invention

The invention relates to a device and kit for introducing at least one physiologically active compound into an organ or cavity of a human or animal body. The device comprises at least one silk material and is loaded with the physiologically active compound.

Gold standard in the treatment of many diseases is the oral administration or injection of a pharmaceutical product on an oftentimes-α-day basis that is poorly accepted by the patients. It is a further disadvantage of conventional rapid-release dosage forms that they predominantly result in high initial drug concentrations in the blood which are not sustained over a longer period of time and that the risk of excess dosing is increased. Therefore, the incidence of side-effects increases and patient's compliance decreases and causes most of the time an interruption of the treatment. Consequently, sustained-release drug delivery systems have been established which allow for maintaining blood serum levels of the drug at a therapeutically effective value and preventing excess dosing and side-effects. Therefore, sustained-release delivery systems increase patient's compliance as patients are less likely to miss a dose with less frequent administration. Less frequent administration, particularly a once-α-day dosage regimen, also increases patient convenience, especially if the treatment requires injection of the drug or even catheterization.

### Prior art

Often, polymer-based systems have been used for sustained-release drug delivery, including the use of a variety of biodegradable and non-biodegradable polymer microparticles, e.g. poly-lactide-co-glycolide particles, containing the active ingredient. Furthermore, various polymer microcapsules and larger sustained-release polymer devices have been used to deliver pharmaceutically active compounds to patients over an extended period of time. However, organic solvents are necessary to prepare polymer-based sustained delivery systems and may cause immunological reactions or anaphylactic shock.

As a consequence of these problems, alternative and more biocompatible drug delivery systems have been developed. For example, EP 2 253 336 A1 discloses a medical device for the treatment of wounds, which comprises a silk protein material loaded with an antimicrobial compound. The device is prepared by impregnating the silk protein material with the antimicrobial compound and drying of the resulting impregnated silk protein material. The silk medical device is used for the treatment of a wound, either treatment of an already infected wound or as a preventive measure, by placing the device on the wound present in the skin of a wounded subject.

However, if a sustained-release drug delivery system has to be introduced into an organ or cavity of a human or animal body, it has to be ensured that the device is either biodegradable or can be easily removed from the organ or cavity after treatment. Further, in special applications, e.g. treatment of diseases of the bladder, it might also be necessary to ensure that the medical device is not excreted from the organ or cavity by way of natural processes such as urination or ulceration.

DE 10 2008 009 646 A1 discloses a medical device for intravesical administration of pharmaceutical compounds for treating diseases of the bladder such as overactive bladder, bladder infections and bladder cancer. The device comprises a plurality of carrier particles which are connected to each other by a bipartite thread, the carrier particles being loaded with a pharmaceutical compound, e.g. Oxybutinin or Trospium chloride. Each part of the bipartite thread is employed as a loop, wherein the thread of the lower loop is fed through the upper loop and extends beyond the end of the catheter used to introduce the chain-like device into the bladder. The device is pressed from the catheter into the lumen of the bladder by means of a pusher and expanded by pulling the thread of the lower loop to form a sphere-like implant. The bulky shape of this sphere-like implant ensures that the implant cannot enter the urethra and thus be rinsed out of the bladder during urination. Accordingly, the implant remains within the lumen of the bladder during the whole treatment period. In order to remove the implant (carriers and threads) from the bladder, the implant can either be biodegradable as a whole or partially rinsed out of the bladder by urination due to sustained degradation of the thread. However, particularly the handling of the threads and the expansion of the device within the bladder is obviously difficult and prone to defects.

### Summary of the invention

It is the object of the invention to provide a device for introducing at least one physiologically active compound into an organ or cavity of a human or animal body, which allows for sustained-release drug delivery while being easy to use and excretable.

The object is achieved by providing a device of the initially mentioned kind wherein the silk material comprises at least one cleavage site that is cleavable by at least one degrading agent. Advantageously, the incorporation of one or more cleavage sites in the silk material allows for controlled degradation of the device. By introduction of defined cleavage sites as predetermined points for breakage of the silk material, it is possible to predetermine the time course and extend of the degradation so as to easily adapt the device according to the invention to specific applications. In particular, determining the number of cleavage sites and their respective position within the silk material, allows for effective control of degradation of the device according to the invention. It is a further advantage of the present invention that the naturally occurring protease activity may be used for the controlled destruction of the silk material into smaller elements. Through continued proteolysis those smaller elements will be eventually small enough to allow facile excretion via natural excretion ways.

As used herein, the term "silk material" refers to any silk protein comprising material such as, e. g., silk protein - based hydrogels, fibers, films, foams, meshes, or multi-layered materials. The term "silk protein" comprises silkworm fibroin and insect or spider silk protein. Preferably, fibroin is obtained from a solution containing a silkworm silk. The silkworm silk protein is obtained, for example, from Bombyx mori. Alternatively, the silk proteins suitable for use in the present invention can be obtained from a solution containing a genetically engineered silk, such as from bacteria, yeast, mammalian cells, transgenic animals or transgenic plants.

The physiologically active compound may be any agent including but not limited to proteins, peptides, nucleotides, polynucleotides, carbohydrates, fatty acids, vesicles, cells, and any combination thereof. Basically, all substance families are suitable for employing the present invention as long as the specific compound exhibits a physiological effect. Preferably, the physiologically active compound may be a pharmaceutical product for treating a human or animal disease.

In a preferred embodiment of the invention, the cleavage site comprises at least one weakened region of the silk material. One or more cleavage sites may be introduced in the silk material by weakening one or more specific regions by means of chemical pretreatment or insertion of at least one molecule that renders the specific region vulnerable for the degrading agent. For example, insertion of one or more amino acids in the silk protein may increase the vulnerability of the respective region to degradation. Preferably, the cleavage sites are introduced in the silk material by insertion of silk material that is thinner than the non-weakened silk material. By determining the number of cleavage sites and/or weakened regions and their respective position within the silk material, degradation of the device according to the invention can be effectively controlled.

Accordingly, it is a preferred embodiment of the invention that the thickness of the silk material within the weakened region(s) is lower than the thickness of the remaining silk material. That is, the degradation of the silk material into small fragments can be effectively controlled through the variation of silk material thickness.

In a particularly preferred embodiment of the invention, the degrading agent comprises at least one proteolytic enzyme, preferably at least one urinary protease. Silk fibroin materials used in this invention are susceptible to complete degradation into very small peptide fragments and amino acids. For example, complete degradation can be achieved with the serine protease subtilisin (Bacillus subtilis) and the protease XIV (Streptomyces griseus). The degrading agent may be inherently present in the target organ or cavity or introduced into the target organ or cavity by the patient or a physician. The degrading agent may alternatively be immobilized on or in the silk material. However, a recent study by Kania et al. (Ann Clin Biochem 2010; 47: 151-157) demonstrated the presence of active proteases in human urine even after storage of patient's samples for 12 months at -20°C. The best characterized urinary protease, which can be easily isolated from human urine, is the serine protease urokinase. The present invention preferably employs the intrinsic protease activity in the urinary bladder for the controlled destruction of the silk material. The speed of degradation can be for example controlled through the thickness of the silk material as described above.

In another preferred embodiment of the invention, the physiologically active compound is immobilized on or in a carrier material. For example, biodegradable poly(D,L-lactide-co-glycolide acids) microspheres having a diameter of about 1 mm are used to immobilize the compound to be delivered. However, any other biocompatible carrier material (degradable or non-degradable) may be used according to the invention. Preferably, the carrier material allows for sustained release of the physiologically active compound so as to ensure maintaining of blood serum levels of the compound at a therapeutically effective value for a longer period of time and prevent excess dosing.

In a further preferred embodiment of the invention, the physiologically active compound and/or the carrier material is/are immobilized on or in the silk material. Preferably, the physiologically active compound, either immobilized or unbound, is physically embedded in the silk material.

In another preferred embodiment of the invention, the silk material is formed by at least one silk film and the physiologically active compound and/or the carrier material is/are embedded in the silk film. By combining silk films having different thickness, e.g. 15 µm and 70 µm, cleavage sites comprising weakened regions may be easily introduced in the silk material so as to control sustained degradation of the silk material.

The silk material may form a ring-shaped silk unit embedding the physiologically active compound and/or the carrier material. Such ring-shaped units are particularly suited to hold a sufficient amount of the physiologically active compound and can be easily handled and introduced into the target organ or cavity.

Preferably, the physiologically active compound comprises a therapeutic agent, in particular a therapeutic agent selected from the group consisting of anticholinergic agents, preferably Trospium chloride, chemotherapeutic agents, antibiotics, anti-viral agents, analgesic agents, and anti-inflammatory agents. Basically, all therapeutic agents suitable for treating diseases of the bladder, in particular anticholinergic drugs such as Trospium chloride, Oxybutinin and Sulifenacin, are preferably used as physiologically active compound in the present invention. The physiologically active compound may also be selected from the group consisting of Trospium chloride, Oxybutinin, Sulifenacin , Tolterodin, Phenoxybenzamin-HCl, Darifenacin, Propiverin, Flavoxat-HCl, Fesoterodinfumarate, Methantheliniumbromide, and Duloxetin.

Accordingly, a preferred application of the device according to the invention is its use for treating medical conditions of the bladder, preferably overactive bladder, urinary incontinence and bladder cancer. Advantageously, the intrinsic protease activity in the urinary bladder can be used for the controlled degradation of the silk material. It is a further main advantage of the present invention that a treatment of bladder diseases using the device according to the invention can be employed with less frequent administration, e.g. at a once-α-month dosage regimen, so that dosage forms at a daily basis or catheterization is avoided.

The present invention further comprises a method for manufacturing the device according to the invention, comprising the steps of:
- providing at least one silk material,
- forming a silk unit comprising the silk material, said silk unit being capable of embedding at least one physiologically active compound and/or at least one carrier including the physiologically active compound,
- introducing at least one cleavage site in the silk material, and
- loading the silk unit with the physiologically active compound and/or the carrier including the physiologically active compound.

In this method, the cleavage site may be introduced in the silk material by inserting at least one amino acid, preferably a sequence of consecutive amino acids, that is specifically recognized by at least one degrading agent.

Preferably, the cleavage site is introduced in the silk material by reducing the thickness of the silk material within at least one specific region as described above.

The object of the invention is also achieved by providing a kit for treating medical conditions of an organ or cavity of a human or animal body, comprising at least one sterilized device according to the invention and means for introducing the device into the organ or cavity.

Alternatively, a kit for treating medical conditions of an organ or cavity of a human or animal body is provided, which comprises at least one sterilized device according to the invention being devoid of the physiologically active compound and means for introducing the device into the organ or cavity. Optionally, this kit further comprises at least one physiologically active compound.

The invention is further exemplarily described in detail with reference to the figures and examples.

### Brief description of the figures

- **Figure 1**: shows a diagram showing release kinetics of Trospium chloride (TrCl) from extrudates based on different amounts of Resomer RG 502H and Resomer RG 502S in water.
Drug loading: 30% TrCl.
(n = 3, mean ± s, H = RG 502H, S = RG 502S)
- **Figure 2**: shows a diagram showing release kinetics of different amounts of Trospium chloride (TrCl) from extrudates based on Resomer RG 502S in water.
(n = 3, mean ± s).
- **Figure 3**: shows a schematic representation demonstrating the degradation of a ring-shaped silk material into smaller elements via proteolysis.
- **Figure 4**: shows photographs demonstrating the control of proteolytic digestion by thickness of silk film.
- **Figure 5**: shows a diagram showing the time course of proteolytic digest (membrane area [%] as a function of time).
- **Figure 6**: shows a drawing showing a a plastic block for manufacturing a device according to the invention.
- **Figure 7**: shows a photograph showing an embodiment of a device according to the invention (Diameter: 71.5 mm, 56 drug carriers).
- **Figure 8**: shows a photograph showing another embodiment of a device according to the invention (Diameter: 24.5 mm, 18 drug carriers).

### Description of exemplary and preferred embodiments of the invention

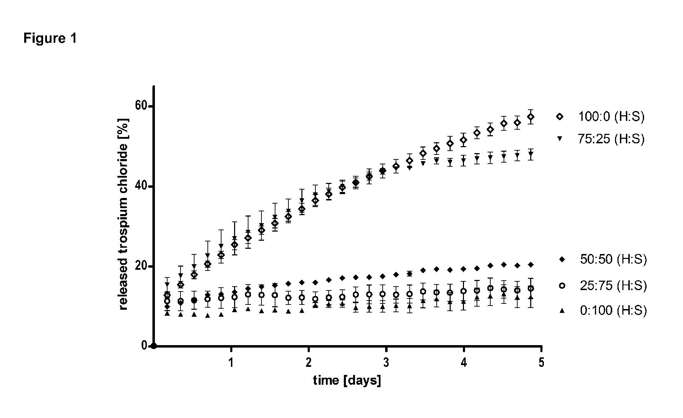

**Figure 1** shows the release [%] of Trospium chloride (TrCl) from drug carriers as a function of time. TrCL was embedded in two different biodegradable poly(D,L-lactide-co-glycolide acids) carriers (Resomer RG 502H and Resomer RG 502S, Evonik). The drug and the carrier were coprocessed via hot-melt extrusion producing extrudates of 1 mm diameter and 2 mm length. Five formulations with different polymer ratios and a drug load of 30% TrCl as well as three formulations with different amounts of TrCl and RG 502S as matrix former were produced. Drug release was investigated for 5 days in USP apparatus I using 895 mL of purified water as dissolution medium. Extrudates consisting of 70% RG 502H and 30% TrCl showed a drug-release of 57 ± 1.8% (mean ± s) within 5 days. Using 70% RG 502S and 30% TrCl resulted in a prolonged drug release of 12 ± 3.0% and is suitable for a long-term application. Further investigations dealt with combinations of both Resomers in order to tailor drug release. Mixtures of RG 502H and RG 502S led to a faster drug release, compared to pure RG 502S as matrix former and a lower release rate than RG 502H. A mixture of 25% H and 75% S led to a drug release of 15 ± 2.5% after 5 days. Increasing the RG 502H amount to 50% and 75% was associated with a faster release within 5 days of 20 ± 0.4% and 48 ± 1.4%, respectively.

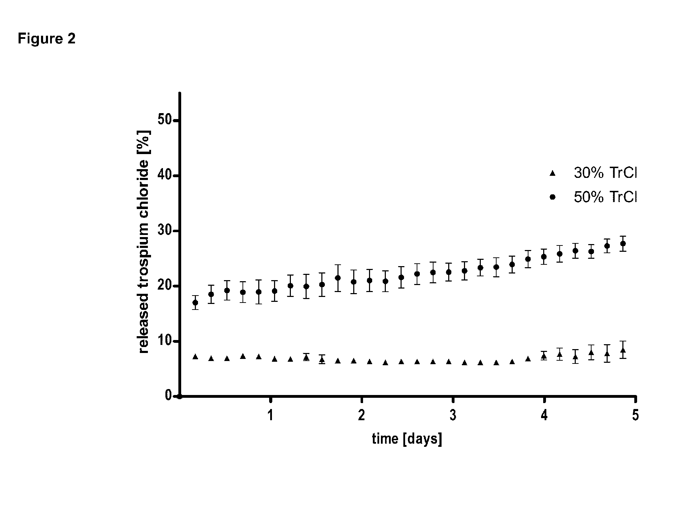

**Figure 2** shows the release [%] of Trospium chloride (TrCl) from drug carriers as a function of time. The maximum drug loading with a prolonged release of TrCl was identified using RG 502S as matrix former. Increasing the drug loading from 30% to 50% led to a faster but still controlled release of TrCl (after 5 days 28 ± 1.4%). A further increase of the API proportion up to 75% caused a complete drug release within a few hours.

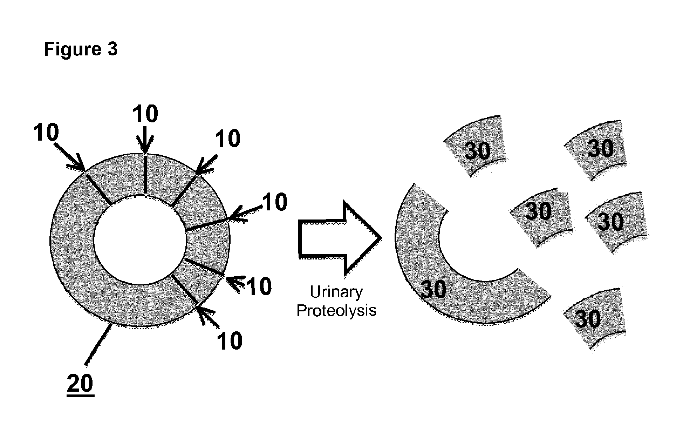

**Figure 3** demonstrates the degradation of a ring-shaped silk material into smaller elements. This figure shows as example the incorporation of cleavage sites 10 in the form of thinner regions compared to the ring-shaped silk material 20. By choice of appropriate thickness as predetermined points for breakage of the silk material, this allows the use of the naturally occurring urinary protease activity for the controlled destruction of the silk ring carrier into smaller elements 30. Through continued urinary proteolysis those smaller elements 30 will be eventually small enough to allow facile discharge via the human urinary tract.

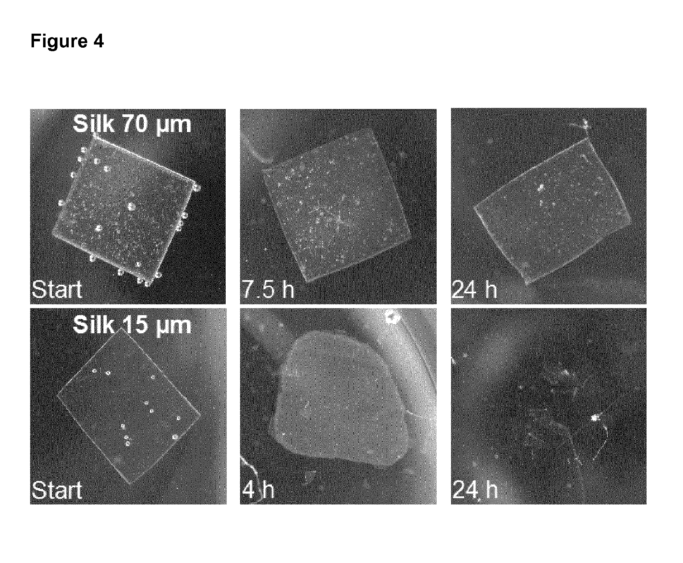

**Figure 4** demonstrates how thickness controls the speed of proteolytic digestion. Silk films were prepared with thickness 15 µm and 70 µm were prepared as described in Example 1. After casting, the silk films were cut into samples with 2x2 mm size and incubated in 1:100 diluted subtilisin protease solution (Sigma Aldrich) at 37°C. The samples were photographically analysed. Figure 4 shows that the silk film sample with thickness 70 µm remains intact after 24 h incubation in subtilisin. In contrast, the silk film with thickness of 15 µm shows strong signs of degradation already after 4 h. After 24 h the 15 µm silk film is entirely degraded into very small fragments. This experiment demonstrates that the speed of proteolytic digestion of an intact silk film into small fragments can be controlled through the variation of silk film thickness.

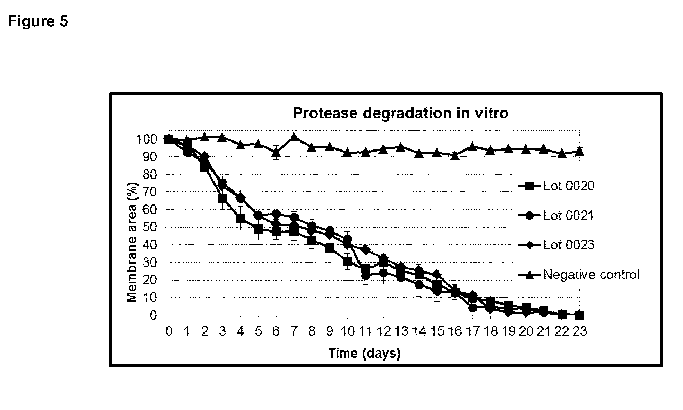

**Figure 5** demonstrates proteolytic digestion of silk films prepared according to the invention. Silk protein solution and silk films (thickness 50 µm) were prepared as described in Example 1. After casting, the silk films were cut into samples with 6 mm diameter and dyed with Neutral Red to support visualization and data analysis with image software (image j). Silk film samples out of Lot 0020, 0021 and 0023 were incubated at 37°C in protease solution (Type XIV 0,93 U/mg Sigma Aldrich), diluted 1:1 in 10 mM sodium and 5 mM calcium acetate solution. Protease XIV is a mixture of at least three caseinolytic activities and one aminopeptidase activity. The caseinolytic enzymes are Streptomyces griseus Protease A, Streptomyces griseus Protease B and the protease Streptomyces griseus Trypsin. The control samples were incubated in sodium acetate solution without protease. The protease solution was changed every day. The membrane area remaining after proteolysis was determined via pixel visualisation as % of original area at the start of the proteolytic digest (t = 0). Figure 5 summarises the results of the proteolytic digest of silk film samples with 50 µm thickness and 6 mm diameter. The silk materials incubated with protease were fully degraded after 23 days. The negative control samples without protease showed no sign of degradation and remained fully intact.

### Example 1

A silk protein solution was extracted from Bombyx mori silkworms via the extraction apparatus described in detail in EP 1 989 223 B1. In brief, silkworm glands of Bombyx mori silkworms at the end of their 5th instar were extracted by removing the silkworm gland from the body of the silkworms. Each of the silkworm glands was cut into half. The posterior halves of the silkworm glands were placed with a pair of forceps in the apparatus for extraction and collection of the silk gland content. After overnight incubation, the extracted silk protein solution was harvested from the extraction apparatus using a disposable syringe.

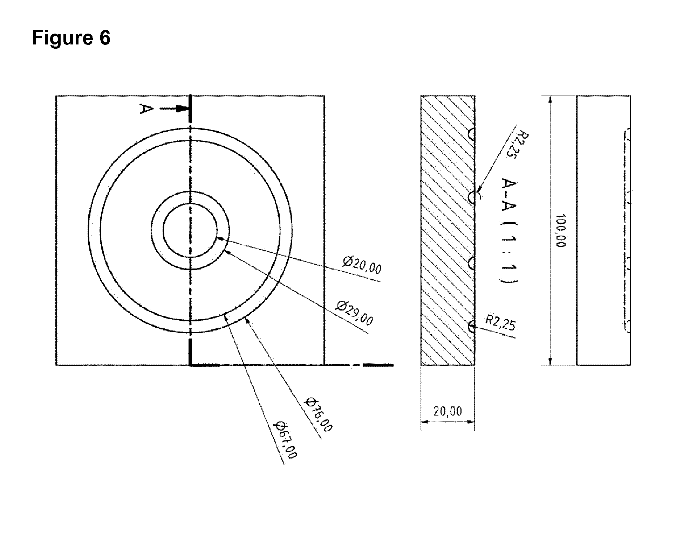

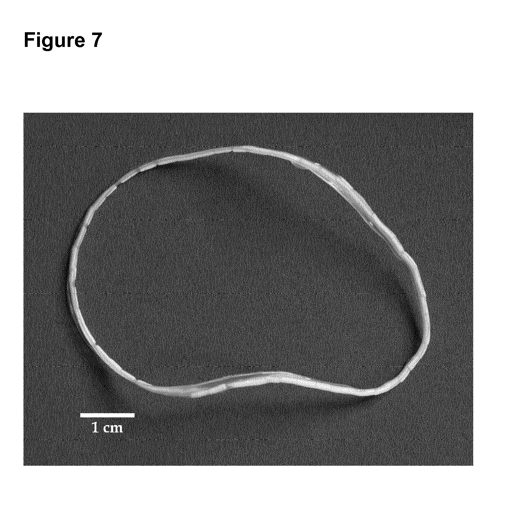

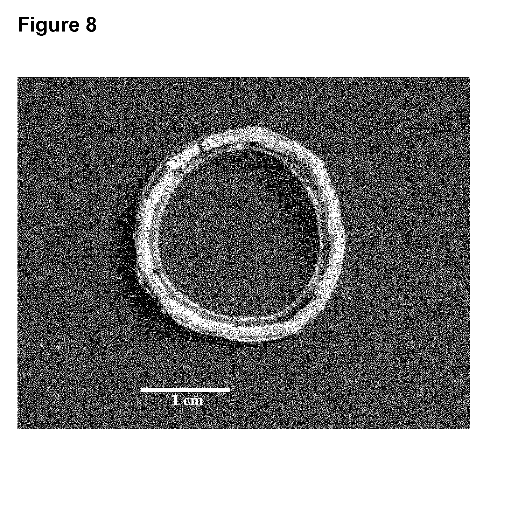

A casting apparatus was manufactured from a plastic block consisting of polytetraflouroethylene (PTFE). Two ring shaped cavities with different diameters were incorporated into the plastic block (**Figure 6**). The cavities were loaded with drug carriers of 2 mm in length and a diameter of 1 mm. The cavity with a mean diameter of 71.5 mm was filled with 56 drug carriers consisting of RG 502 S and 50% Trospium chloride, the cavity with a mean diameter of 24.5 mm was loaded with 18 drug carriers consisting of RG 502 H and 30% Trospium chloride. Afterwards the silk protein solution was applied to the prefilled casting apparatus. After drying overnight, the cast silk-drug carrier-ring was harvested from the casting apparatus manually. The embedded drug carriers were clearly visible though the transparent silk structure (**Figures 7** **and** **8**).

## Claims

1. Device for introducing at least one physiologically active compound into an organ or cavity of a human or animal body, said device comprising at least one silk material and being loaded with the physiologically active compound, wherein the silk material comprises at least one cleavage site that is cleavable by at least one degrading agent.

2. Device according to claim 1, wherein the cleavage site comprises at least one weakened region of the silk material.

3. Device according to claim 2, wherein the thickness of the silk material within the weakened region(s) is lower than the thickness of the remaining silk material.

4. Device according to claim 1, 2 or 3, wherein the degrading agent comprises at least one proteolytic enzyme, preferably at least one urinary protease.

5. Device according to any one of claims 1 to 4, wherein the physiologically active compound is immobilized on or in a carrier material.

6. Device according to any one of claims 1 to 5, wherein the physiologically active compound and/or the carrier material is/are immobilized on or in the silk material.

7. Device according to any one of claims 1 to 6, wherein the silk material is formed by at least one silk film and the physiologically active compound and/or the carrier material is/are embedded in the silk film.

8. Device according to any one of claims 1 to 7, wherein the silk material forms a ring-shaped silk unit embedding the physiologically active compound and/or the carrier material.

9. Device according to any one of claims 1 to 8, wherein the physiologically active compound comprises a therapeutic agent, in particular a therapeutic agent selected from the group consisting of anticholinergic agents, chemotherapeutic agents, antibiotics, anti-viral agents, analgesic agents, and anti-inflammatory agents, preferably Trospium chloride.

10. Use of the device according to any one of claims 1 to 9 for treating medical conditions of the bladder, preferably overactive bladder, urinary incontinence and bladder cancer.

11. Method for manufacturing the device according to any one of claims 1 to 9, said method comprising:
a) providing at least one silk material,
b) forming a silk unit comprising the silk material, said silk unit being capable of embedding at least one physiologically active compound and/or at least one carrier including the physiologically active compound,
c) introducing at least one cleavage site in the silk material, and
d) loading the silk unit with the physiologically active compound and/or the carrier including the physiologically active compound.

12. Method according to claim 11, wherein the cleavage site is introduced in the silk material by inserting at least one amino acid, preferably a sequence of consecutive amino acids, that is specifically recognized by at least one degrading agent.

13. Method according to claim 11, wherein the cleavage site is introduced in the silk material by reducing the thickness of the silk material within at least one specific region.

14. A kit for treating medical conditions of an organ or cavity of a human or animal body, comprising at least one sterilized device according to any one of claims 1 to 9 and means for introducing the device into the organ or cavity.

15. A kit for treating medical conditions of an organ or cavity of a human or animal body, comprising at least one sterilized device according to any one of claims 1 to 9 being devoid of the physiologically active compound and means for introducing the device into the organ or cavity, and optionally at least one physiologically active compound.
